# EUROPÄISCHE PATENTANMELDUNG

(11) **EP 4 704 097 A1**
(43) Veröffentlichungstag der Anmeldung: **04.03.2026**
(21) Anmeldenummer: 24197903.8
(22) Anmeldetag: 02.09.2024
(51) Int. Cl.: G16H 10/60

(54) **VERFAHREN ZUR ANONYMEN AUSWERTUNG VON SICHERHEITSKRITISCHEN DATENSÄTZEN**

(71) Anmelder: Research Industrial Systems Engineering (RISE) Forschungs-, Entwicklungs- und Grossprojektberatung GmbH, 2320 Schwechat (AT)
(72) Erfinder: SCHÖNBAUER, Franz, 1040 Wien (AT); GRECHENIG, Thomas, 1040 Wien (AT)
(74) Vertreter: Schwarz & Partner Patentanwälte GmbH

(57) **Zusammenfassung**

Die Erfindung betrifft ein computerimplementiertes Verfahren zur anonymen Bereitstellung von sicherheitskritischen Datensätzen (DSi), insbesondere von gesundheitsbezogenen Datensätzen, umfassend die folgenden, in einem Rechenzentrum (2) durchgeführten Schritte:
- Speichern einer Vielzahl von sicherheitskritischen Datensätzen (DSi), die jeweils mehrere Dateneinträge (valj), die insbesondere medizinische Messdaten sind, umfassen,
- über eine externe Schnittstelle, Empfangen eines Computerprogramms (exel, exe2, </3>), das dazu ausgebildet ist, bei dessen Ausführung ausgewählte Dateneinträge (valj) aus den Datensätzen (DSi) zu extrahieren und/oder zu verarbeiten, wobei die extrahierten oder verarbeiteten Dateneinträge (valj) in zumindest einem Ausgabedatensatz (5) gespeichert werden,
- Ausführen des Computerprogramms (exel, exe2, </3>); und
- Ausgeben des zumindest einen Ausgabedatensatzes (5) über die genannte oder eine andere externe Schnittstelle.

## Beschreibung

Die Erfindung betrifft ein Verfahren zur anonymen Auswertung von sicherheitskritischen Datensätzen.

Gesundheitsbezogene Datensätze umfassen eine Vielzahl sensibler Informationen, die sich auf den physischen und psychischen Zustand von Individuen beziehen. Diese Daten können unter anderem medizinische Anamnesen, Diagnoseberichte, Behandlungsverläufe, Laborergebnisse und genetische Informationen enthalten. Aufgrund ihrer Sensibilität und des Potenzials für Missbrauch ist der Schutz und die sichere Verarbeitung dieser Daten von entscheidender Bedeutung.

Die sicherheitskritische Verarbeitung gesundheitsbezogener Datensätze erfordert den Einsatz fortschrittlicher Technologien und Methoden, um den unbefugten Zugriff, die Manipulation oder den Verlust von Daten zu verhindern. Zu den wesentlichen Sicherheitsmaßnahmen gehören die Verschlüsselung der Daten sowohl bei der Übertragung als auch im Ruhezustand, die Implementierung von Zugriffskontrollen basierend auf Rollen und Berechtigungen sowie die kontinuierliche Überwachung und Protokollierung von Zugriffen und Aktivitäten. Es kann zusammengefasst werden, dass die sichere Aufbewahrung gesundheitsbezogener Datensätze robuste und zuverlässige Infrastrukturen erfordert, die sowohl physische als auch digitale Sicherheitsmaßnahmen umfassen.

Weiterhin sind ethische Überlegungen bezüglich der Nutzung und Weitergabe dieser Daten von zentraler Bedeutung, insbesondere im Hinblick auf die Minimierung von Risiken für die betroffenen Individuen. Die Entwicklung und Implementierung von Lösungen zur sicheren Handhabung gesundheitsbezogener Datensätze muss daher stets im Einklang mit diesen gesetzlichen und ethischen Anforderungen stehen, um das Vertrauen der Öffentlichkeit und der betroffenen Personen zu gewährleisten.

Die Weitergabe gesundheitsbezogener Datensätze an Dritte, beispielsweise für Studienzwecke oder statistische Auswertungen, birgt zahlreiche Herausforderungen. Eine der größten technischen Herausforderungen besteht darin, sicherzustellen, dass die Daten anonymisiert oder pseudonymisiert werden, um die Identität der betroffenen Personen zu schützen. Dies erfordert spezielle Techniken, die verhindern, dass individuelle Daten durch Rückschlussverfahren reidentifiziert werden können. Gleichzeitig muss die Datenintegrität gewahrt bleiben, sodass die für die Analyse relevanten Informationen vollständig und korrekt bleiben. Ein weiteres Problem stellt die Sicherstellung der Datenqualität dar, da nur qualitativ hochwertige und konsistente Daten verlässliche Ergebnisse liefern können.

Die Weitergabe gesundheitsbezogener Datensätze an Dritte kann beispielsweise auch deshalb erfolgen, da nur wenige Dritte (z.B. Google, Meta, Amazon etc.) über große Rechenzentren zur Auswertung gesundheitsbezogener Datensätze mittels Künstlicher Intelligenz (KI) besitzen. Zwar bietet die Auswertung gesundheitsbezogener Datensätze mittels KI erhebliche Potenziale zur Verbesserung der medizinischen Forschung und der patientenbezogenen Versorgung, jedoch ist es aus den vorgenannten Gründen nicht wünschenswert, dass die Datensätze an diese an die genannten Dritten übergeben werden.

Im Stand der Technik wurden die Datensätze bislang anonymisiert oder pseudonymisiert und als Ganzes an die Dritten zur Auswertung weitergegeben.

Die vorliegende Erfindung wurde somit vor dem Hintergrund gemacht, dass die Weitergabe von sicherheitskritischen Datensätzen hohe Sicherheitsvorkehrungen erfordert, um den Schutz dieser Daten bei der Weitergabe und während der Verarbeitung zu gewährleisten und das Risiko von Datenlecks oder Missbrauch zu minimieren.

Es ist ein Ziel der Erfindung, Dritten sicherheitskritische Datensätze, insbesondere von gesundheitsbezogene Datensätzen, zu übergeben, damit diese von den Dritten ausgewertet werden können. Gleichzeitig soll jedoch hohe Sicherheitsmaßnahmen vorgesehen werden.

Dieses Ziel wird durch ein Verfahren zur anonymen Bereitstellung von sicherheitskritischen Datensätzen, insbesondere von gesundheitsbezogenen Datensätzen, gelöst, wobei das Verfahren die folgenden, in einem Rechenzentrum durchgeführten Schritte umfasst:
- Speichern einer Vielzahl von sicherheitskritischen Datensätzen, die jeweils mehrere Dateneinträge von Daten, insbesondere medizinischen Messdaten, umfassen,
- über eine externe Schnittstelle, Empfangen eines Computerprogramms, das dazu ausgebildet ist, bei dessen Ausführung ausgewählte Dateneinträge aus den Datensätzen zu extrahieren und/oder zu verarbeiten, wobei die extrahierten oder verarbeiteten Dateneinträge in zumindest einem Ausgabedatensatz gespeichert werden,
- Ausführen des Computerprogramms; und
- Ausgeben des zumindest einen Ausgabedatensatzes über die genannte oder eine andere externe Schnittstelle.

Die vorliegende Erfindung ermöglicht, dass die vollständigen Datensätze innerhalb des Rechenzentrums verbleiben können, und nicht als Ganzes an Dritte zur Auswertung übergeben werden müssen. Unter "hierin beschriebenen Rechenzentrum" wird ein Rechenzentrum verstanden, das sicherheitskritische Datensätze speichert, die nicht öffentlich zugänglich sind. Das Rechenzentrum könnte nur durch eine einzige Recheneinheit gebildet sein oder durch mehrere Recheneinheiten.

Es ist eine Erkenntnis der Erfindung, dass für eine gezielte Auswertung der Datensätze nicht immer alle Dateneinträge der Datensätze notwendig sind (und daher einige Dateneinträge extrahiert werden können) bzw. dass die Dateneinträge vorverarbeitet werden können, bevor diese an den Dritten bereitgestellt werden.

Eine weitere Erkenntnis bei der erfindungsgemäßen Lösung ist, dass nicht durch den Betreiber des Rechenzentrums vorab bestimmt werden kann, welche Ausgabedatensätze zur Untersuchung einer medizinischen Eigenschaft relevant sein könnten. In anderen Worten obliegt es nicht dem Betreiber des Rechenzentrums, die Auswahl der Ausgabedatensätze für eine bestimmte medizinische Eigenschaft vorzunehmen. Diese Auswahl wird durch Forscher von Universitäten oder Entwickler von Dritten vorgenommen und dem Betreiber des Rechenzentrums in Form des Computerprogramms mitgeteilt. Durch die Ausführung des Computerprogramms innerhalb des Rechenzentrums und damit die interne Zusammenstellung des Ausgabedatensatzes muss den Forschern oder den Entwicklern nicht der gesamte Datensatz bereitgestellt werden.

Durch die erfindungsgemäße Lösung kann verhindert werden, dass die gesamten Datensätze den Dritten zur Verfügung gestellt werden müssen, damit diese sich die benötigten Daten selbst aus den Datensätzen extrahieren können. Bei der erfindungsgemäßen Lösung wird somit nur eine Teilmenge der Datensätze an die Dritten bereitgestellt, was es erheblich erschwert, diese mit anderen Datensätzen zu korrelieren und damit bestimmten Personen zuzuordnen. Gleichzeitig bleibt es jedoch den Dritten überlassen, welche Daten - insbesondere in welcher verarbeiteten Form - für die Forschungsarbeiten benötigt werden.

Beispielsweise könnten im Rechenzentrum Blutbefunde als Datensätze gespeichert sein, wobei die einzelnen Blutbefunde jeweils mehrere Blutwerte, d.h. medizinische Messdaten, umfassen. Die Blutwerte können beispielsweise zumindest zwei der folgenden Werte sein: Hämoglobin (Hb), Hämatokrit (Hkt), Erythrozyten (rote Blutkörperchen, RBC), Leukozyten (weiße Blutkörperchen, WBC), Thrombozyten (Blutplättchen, PLT), Blutglukose, Kreatinin, Cholesterin, Elektrolyte, C-reaktives Protein (CRP). Würde der gesamte Datensatz ausgegeben werden, wäre es aufgrund der Vielzahl an Dateneinträgen leicht möglich, diesen mit einem weiteren Datensatz korrelieren, der gegebenenfalls zusammen mit anderen Daten ausgegeben worden ist und "geleakt" wurde. Wenn nun Forscher oder Entwickler zur Bestimmung einer bestimmten medizinischen Eigenschaft, wie der Wahrscheinlichkeit eines bestimmten Krankheitsverlauf, nur zwei der genannten Blutwerte wie Hb und WBC benötigen, aber nicht die restlichen Blutwerte, kann das Computerprogramm derart ausgestaltet sein, dass nur diese zwei Blutwerte als extrahierte Dateneinträge im Ausgabedatensatz gespeichert werden. Da der Ausgabedatensatz gegenüber dem ursprünglichen Datensatz stark eingeschränkt ist, ist es in der Regel nicht möglich, den Ausgabedatensatz mit dem ursprünglichen Datensatz zu korrelieren (da der Ausgabedatensatz gegebenenfalls vielen Datensätzen zugeordnet werden kann). Weiters besteht der Vorteil, dass zum Zwecke einer weiteren Forschung dasselbe Verfahren durchgeführt wird und mit dem genannten Verfahren wieder nur ein (verarbeiteter) Unterdatensatz ausgegeben wird, wodurch alle vom Forschungszentrum ausgegebenen Ausgabedatensätze aufgrund der wenigen überlappenden Datenwerte viel schwerer miteinander korrelierbar sind.

Noch vorteilhafter ist es, wenn der Ausgabedatensatz nur verarbeitete Dateneinträge umfasst. Im vorherstehenden Beispiel könnten für die Forscher beispielsweise nur die Differenzen (Hb - WBC) und (RBC - PLT) relevant sein, wobei das Computerprogramm derart ausgestaltet sein kann, dass nur diese zwei Differenzen als verarbeitete Dateneinträge im Ausgabedatensatz gespeichert werden. Dadurch kann nicht mehr oder nur äußerst schwer nachvollzogen werden, welcher Ausgabedatensatz zu welchem Datensatz zugehörig ist. Im Allgemeinen wird bevorzugt, wenn der Ausgabedatensatz beim Verarbeiten von Dateneinträgen (valj) zumindest zwei Dateneinträge (valj) eines Datensatzes (DSi) herangezogen werden und aus diesen ein Wert berechnet wird, der im Ausgabedatensatz (5) gespeichert wird. Der Ausgabedatensatz kann zumindest einen oder bevorzugt nur derartige Werte berechnen, die aus zumindest zwei Dateneinträgen errechnet wurden.

Eine weitere Möglichkeit, Dateneinträge verarbeitet im Ausgabedatensatz zu speichern, ist, dass diese gemäß dem Computerprogramm verrauscht werden und danach z.B. zufällig im Bereich von +/- 1% des ursprünglichen Wertes liegen. Weitere Verfahren zum Vorverarbeiten von Dateneinträgen sind möglich.

Es ist weite weitere Erkenntnis der Erfindung, dass das hierin beschriebene Verfahren nicht nur auf gesundheitsbezogene Datensätze angewandt werden kann, sondern auch auf andere sicherheitskritische Datensätze. Beispielsweise kann das Rechenzentrum personenbezogene Datensätze mit Daten wie Verkehrsdelikten, unbezahlte Kreditraten etc. umfassen. Wenn nun eine Exekutivbehörde im Zuge einer ersten Ermittlung wie einer Fahrerflucht Daten des Rechenzentrums benötigt, könnten für diese erste Ermittlung vergangene Verkehrsdelikte, aber nicht die unbezahlten Kreditdaten relevant sein. In diesem Fall ist das Computerprogramm derart ausgeführt, dass im Ausgabesatz nur die vergangenen Verkehrsdelikte im Ausgabedatensatz enthalten sind. Wenn die Exekutivbehörde im Zuge einer zweiten Ermittlung wie eines Steuerbetrugs Daten des Rechenzentrums benötigt, könnten für diese zweite Ermittlung unbezahlte Kreditdaten, aber nicht vergangene Verkehrsdelikte relevant sein. In diesem Fall ist das Computerprogramm derart ausgeführt, dass im Ausgabesatz nur die Kreditdaten im Ausgabedatensatz enthalten sind.

Bei dem vorstehend erläuterten Verfahren ist es insbesondere wichtig, dass transparent ist, dass das Computerprogramm keine unberechtigten Daten in den Ausgabedatensatz ausgibt. In einem ersten Schritt könnte der Betreiber des Rechenzentrums Computerprogramme nur von vertrauenswürdigen Sendern empfangen, die z.B. in einer Liste des Rechenzentrums angeführt sind. Um jedoch nicht nur auf dieser Vertrauensbasis zu arbeiten, kann als zusätzliche Sicherheitsstufe eingeführt werden, dass innerhalb des Rechenzentrums überprüfbar ist, ob das Computerprogramm keine unberechtigten Daten in den Ausgabedatensätzen speichert. Dies kann jedoch nicht oder nicht alleine durch die Kontrolle des Ausgabedatensatzes erfolgen. Aus diesem Grund können eine oder mehrere der folgenden Maßnahmen eingesetzt werden.

In einer ersten bevorzugten Ausführungsform kann das Computerprogramm eine Indikation einer zu untersuchenden Eigenschaft umfassen, insbesondere einer zu untersuchenden medizinischen Eigenschaft, wobei das Computerprogramm dazu ausgebildet ist, im zumindest einen Ausgabesatz nur für die genannte Indikation relevante Daten zu speichern. Unter der Indikation wird verstanden, was der Sender des Computerprogrammes mittels der Ausgabedatensätze vornehmen will, z.B. welchen Krankheitsverlauf dieser untersuchen will oder welches Ermittlungsverfahren dieser führt.

In einer ersten Variante kann das Rechenzentrum damit beispielsweise überprüfen, ob der Sender des Computerprogramms dazu berechtigt ist, Ausgabedatensätze für die genannte Indikation zu erhalten, und nur bei Vorliegen der Berechtigung den Ausgabedatensatz ausgibt. Beispielsweise kann im Rechenzentrum eine Liste geführt werden, in der für vorbestimmte Sender vorbestimmte Indikationen aufgelistet sind. In einem Beispiel darf Google nur an Krebs forschen, aber nicht an Geschlechtskrankheiten; oder eine Finanzbehörde darf nur Steuerverfahren bearbeiten, aber keine Verkehrsdelikte überprüfen. In diesen Fällen könnten "Krebs", "Geschlechtskrankheit" (beides medizinische Indikationen), "Steuerverfahren" und "Verkehrsdelikt" (beides keine medizinischen Indikationen) mögliche Indikationen sein.

Der Betreiber des Rechenzentrums kann auch überprüfen, ob der Ausgabedatensatz mit der Indikation übereinstimmt. Hierfür kann das Rechenzentrum nach dem Schritt des Ausführens und vor dem Schritt des Ausgebens den folgenden Schritt durchführen:
- Überprüfen anhand der Ausgabedaten, ob das Computerprogramm nur auf solche Dateneinträge (valj) aus den Datensätzen (DSi) zugreift oder nur solche ausgewählten Dateneinträge aus den Datensätzen in Ausgabedatensätzen speichert, die zu der genannten Indikation zugehörig sind.

Wenn im Ausgabedatensatz Dateneinträge (extrahiert oder verarbeitet) vorliegen, die nicht mit der Indikation korrelieren, kann das Verfahren gestoppt werden, bevor die Ausgabedatensätze ausgegeben werden. Beispielsweise kann eine Indikation "Geschlechtskrankheit" sein, aber im Ausgabedatensatz eine "Augenfarbe" angeführt sein. Da dieser Datensatz im Ausgabedatensatz nicht zu der Indikation zugehörig ist, wird der Ausgabedatensatz nicht ausgegeben. Beispielsweise kann im Rechenzentrum eine Liste geführt werden, in der für vorbestimmte Indikationen vorbestimmte Datensätze aufgelistet sind. Alternativ oder zusätzlich könnte das Rechenzentrum nur einen Zugriff auf jene Dateneinträge zulässt, die zu der genannten Indikation zugehörig sind (z.B. gemäß der unten erläuterten Liste).

Wie bereits erläutert könnten die Dateneinträge z.B. auch verrauscht oder anders verarbeitet werden, sodass nicht immer nachvollziehbar ist, woher ein Dateneintrag im Ausgabedatensatz stammt. Dies könnte von betrügerischen Sendern von Computerprogrammen ausgenutzt werden, um z.B. das Computerprogramm derart auszuführen, dass dieses einen Hb-Wert heranzieht, verrauscht und als Hkt-Wert ausweist. Für den Betreiber des Rechenzentrums erscheint es, als ob das Computerprogramm Hkt-Werte ausgibt, obwohl tatsächlich Hb-Werte ausgegeben werden, ohne dass das Rechenzentrum dies nachweisen könnte. Durch ein Verarbeiten von mehreren Dateneinträgen sind viele weitere Manipulationstechniken möglich. Um dies zu verhindern, kann der Schritt des Empfangens des Computerprogramms folgende Schritte umfasst:
- Empfangen des Computerprogramms als Quellcode; und
- optional Kompilieren oder maschinelles Interpretieren des Quellcodes; und
Ausführen des kompilierten oder interpretierten Quellcodes als Computerprogramm.

Da das Rechenzentrum in dieser bevorzugten Ausführungsform den Quellcode erhält, können die einzelnen im Quellcode definierten Funktionen überprüft werden. Es kann insbesondere nachvollzogen werden, welche Dateneinträge das Computerprogramm heranzieht und wie verarbeitet. Das Rechenzentrum kann beispielsweise sowohl den Quellcode als auch den bereits kompilierten oder interpretierten Quellcode enthalten. Bevorzugt erhält das Rechenzentrum jedoch nur den Quellcode und kompiliert oder interpretiert diesen selbst, um ihn als Computerprogramm auszuführen.

In einer ersten Variante kann der Quellcode auch nur dazu eingesetzt werden, um zu überprüfen, ob das Computerprogramm keine schadhaften Funktionen im Rechenzentrum durchführt, z.B. indem eine der genannten Listen durch das Computerprogramm verändert wird. Bevorzugt kann das Verfahren jedoch den folgenden vor der Ausführung des Computerprogramms durchgeführten Schritt umfassen:
- Überprüfen anhand des Quellcodes, ob das Computerprogramm dazu ausgebildet ist, bei dessen Ausführung nur solche Dateneinträge aus den Datensätzen in Ausgabedatensätzen zu speichern oder bei dessen Ausführung nur auf solche Dateneinträge aus den Datensätzen zuzugreifen, die zu der genannten Indikation zugehörig sind.

Damit können die oben beschriebenen Manipulationen unterbunden werden, denn es könnte festgestellt werden, dass ein als Hkt-Wert ausgewiesener Wert gemäß den im Quellcode angegebenen Funktionen eigentlich ein Hb-Wert ist.

Um hohe Sicherheitsstandards zu erfüllen, kann vorgesehen werden, dass das Rechenzentrum das Computerprogramm von einem ersten anfragenden Kommunikationspartner empfängt und den Ausgabedatensatz nur an den ersten Kommunikationspartner ausgibt, d.h. der Ausgabedatensatz wird nur diesem Kommunikationspartner zur Verfügung gestellt. Weiters kann Rechenzentrum ein zweites Computerprogramm von einem zweiten Kommunikationspartner empfangen und einen gemäß dem zweiten Computerprogramm erzeugten zweiten Ausgabedatensatz nur an den zweiten Kommunikationspartner ausgeben.

Bevorzugt werden alle oben beschriebenen Schritte vollständig computerimplementiert durch das Rechenzentrum durchgeführt, insbesondere auch die Schritte des Überprüfens, was z.B. anhand der beschriebenen Listen umgesetzt werden kann. Alternativ könnten die Schritte des Überprüfens auch durch menschliches Mitwirken vorgenommen werden, z.B. indem der Person der Quellcode an einem Bildschirm gezeigt wird, sodass dieser den Quellcode überprüfen kann. Das Überprüfen des Quellcodes könnte aber auch vollständig computerimplementiert, z.B. durch eine KI, vorgenommen werden.

In einem weiteren Aspekt betrifft die Erfindung ein Rechenzentrum, welches dazu ausgebildet ist, das genannte Verfahren durchzuführen.

Um die vorliegende Erfindung besser zu veranschaulichen und deren Funktionsweise detailliert zu erklären, wird im Folgenden auf die beigefügte Figur Bezug genommen. Diese Abbildung dient der Verdeutlichung der technischen Merkmale der Erfindung. Die nachfolgende Beschreibung der Figur soll dazu beitragen, die strukturellen und funktionalen Eigenschaften der Erfindung eingehend zu verstehen und deren Vorteile und technischen Fortschritte gegenüber dem Stand der Technik zu erläutern. Darin zeigt:
Figur 1 ein Rechenzentrum, das ein Computerprogramm von einem Teilnehmer empfängt und einen Ausgabedatensatz an diesen retourniert.
Figur 2 zeigt eine Variante von Figur 1, wobei ein Quellcode empfangen wird.

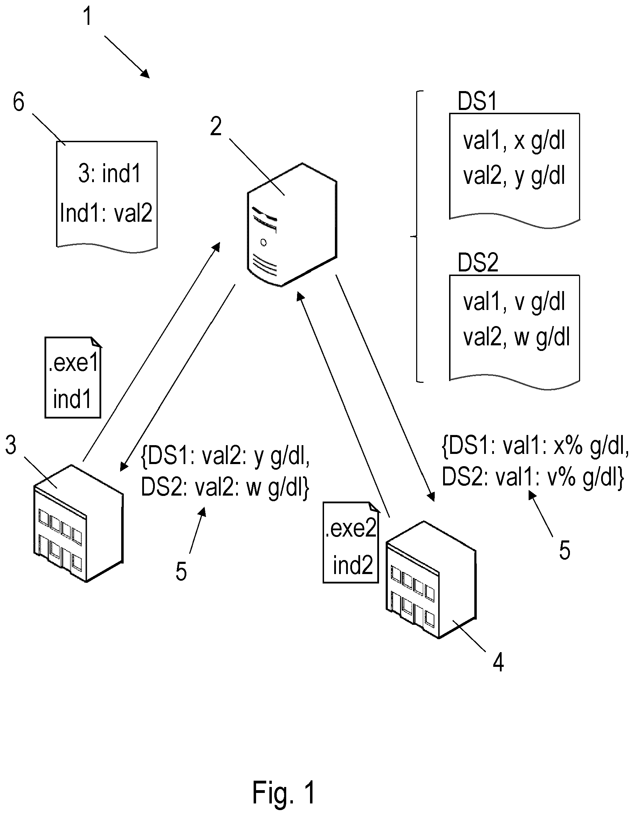

Figur 1 zeigt ein System 1 mit einem Rechenzentrum 2, in dem sicherheitskritische Datensätze DS1, DS2, ..., allgemein DSi, vorliegen. Die sicherheitskritischen Datensätze DSi umfassen jeweils personenbezogene Dateneinträge val1, val2, ... allgemein valj, sodass höchste Sicherheitsanforderungen an das Rechenzentrum 2 gestellt werden. Wenn die Dateneinträge valj medizinische Daten, insbesondere medizinische Messdaten wie Blutwerte sind, spricht man auch von gesundheitsbezogenen Daten.

Üblicherweise wird eine riesige Menge an Datensätzen DSi im Rechenzentrum 1 vorliegen, sodass diese nicht manuell auswertbar sind. Es besteht jedoch ein Bedarf von Forschungseinrichtungen oder dergleichen, diese Daten anonym auszuwerten, z.B. um Studien durchzuführen oder insbesondere um diese riesigen Datenmengen mittels KI auszuwerten. Bislang wurde vorgesehen, die Datensätze DSi zu anonymisieren oder pseudonymisieren und sie den Forschungseinrichtungen in dieser Form zur Verfügung zu stellen, jedoch gelingt es immer wieder, diese Datensätze DSi den Personen zuzuweisen, von denen sie ursprünglich stammen. Um dieses Risiko zu minimieren, wird das im Folgenden beschriebene Verfahren eingesetzt.

Ein erster Kommunikationspartner 3 (auch Sender genannt) erstellt ein Computerprogramm, da er beispielsweise an einer ersten Krankheit forscht. Diese erste Krankheit wird hierin als Indikation ind1 bezeichnet. Im Allgemeinen spricht man auch von einer Indikation einer zu untersuchenden Eigenschaft. Um an dieser Krankheit zu forschen, benötigt der erste Kommunikationspartner 3 beispielsweise die Dateneinträge val2 aus allen Datensätzen DSi. In der Regel wird der erste Kommunikationspartner einen Untersatz von Dateneinträgen valj aller Datensätze DSi benötigen, nie jedoch alle Dateneinträge valj der Datensätze DSi.

Der erste Kommunikationspartner 3 schreibt folglich ein Computerprogramm exe1 (dies wird nur zur Zwecke der Darstellung als ".exe"-Datei bezeichnet, es könnte jegliche andere Dateiendung aufweisen). Der erste Kommunikationspartner 3 sendet das Computerprogramm exe1 an das Rechenzentrum 2. Somit kann das Rechenzentrum 2 das erste Computerprogramm exe1 über eine externe Schnittstelle empfangen. Die externe Schnittstelle kann z.B. eine Internetschnittstelle sein, eine USB-Schnittstelle oder dergleichen. Es soll lediglich ausgeschlossen sein, dass es sich um eine interne Schnittstelle einer Recheneinheit, z.B. zwischen CPU und RAM, handelt.

Sobald das Rechenzentrum 2 das erste Computerprogramm empfangen hat, kann es dieses ausführen. Das Computerprogramm kann hierbei als bereits kompiliertes Computerproramm empfangen werden, wobei dies nicht zwingend ist, siehe die Ausführungen zum Quellcode unten. Wenn das erste Computerprogramm exe1 im Rechenzentrum 2 ausgeführt wird, extrahiert dieses jene ausgewählten Dateneinträge valj aus den Datensätzen DSi, die von ersten Kommunikationspartner 3 zur Untersuchung der genannten Indikation ind1 benötigt werden. Das erste Computerprogramm exe1 speichert diese extrahierten Dateneinträge valj in einem Ausgabedatensatz 5. Es könnte sich auch um mehrere Ausgabedatensätze 5 handeln, was im Folgenden implizit mitumfasst wird.

Im konkreten Beispiel von Figur 1 wurden für die Indikation ind1 alle Dateneinträge "val2" benötigt. Der Ausgabedatensatz 5, der aus dem ersten Computerprogramm exe1 entstand, umfasst somit die Dateneinträge val2 aller Datensätze DSi. Die dargestellte Datenstruktur {DS1: val2: y g/dl, DS2: val2: w g/dl} des Ausgabedatensatzes 5 wurde nur zur Veranschaulichung gewählt. Der Ausgabedatensatz 5 könnte beispielsweise auch die Dateneinträge val4 aller Datensätze DSi umfassen, wenn diese für die Untersuchung der Indikation ind1 benötigt werden.

Danach sendet das Rechenzentrum 2 den Ausgabedatensatz 5 wieder an denselben ersten Kommunikationspartner 3, von dem das erste Computerprogramm exe1 stammte. Es versteht sich, dass hierfür alle modernen Verschlüsselungstechniken eingesetzt werden können. Das Senden des Ausgabedatensatzes 5 erfolgt über dieselbe externe Schnittstelle oder eine andere externe Schnittstelle, z.B. eine Internetschnittstelle sein, eine USB-Schnittstelle oder dergleichen.

Weiters ist ersichtlich, dass mit dem ersten Computerprogramm exe1 auch die Angabe der ersten Indikation ind1 mitgesandt wird, beispielsweise als zusätzliche Datei oder als Teil des Computerprogrammes exe2. Das Rechenzentrum 2 kann dadurch überprüfen, ob der erste Kommunikationspartner 3 überhaupt Ausgabedatensätze 5 für die erste Indikation ind1 erhalten darf. Hierfür kann vorab eine Liste 3 im Rechenzentrum 2 hinterlegt sein, in der für jeden Kommunikationspartner 3, 4 angegeben ist, für welche Indikation ind1, ind2 er Ausgabedatensätze 5 erhalten darf. In Figur 1 ist dies durch "3: ind1" dargestellt, d.h. der Kommunikationspartner 3 darf Ausgabedatensätze 5 für die erste Indikation ind1 erhalten.

Weiters kann in dieser Liste 6 oder in einer anderen Liste hinterlegt sein, welche Dateneinträge valj für eine jeweilige Indikation ind1, ind2 maximal (verarbeitet) ausgegeben werden dürfen. Selbstverständlich kann das Computerprogramm exe1 auch weniger Dateneinträge valj ausgeben lassen, als in der Liste angeführt sind. In Figur 1 darf für die erste Indikation ind1 nur der zweite Dateneintrag val2 ausgegeben werden, siehe "ind1: val2".

Weiters ist aus Figur 1 ein zweiter Kommunikationspartner 4 ersichtlich, der ein zweites Computerprogramm exe2 für eine zweite Indikation ind2 erstellt und an das Rechenzentrum sendet, gegebenenfalls zusammen mit der zweiten Indikation ind2 für die genannten Zwecke der Überprüfung der Berechtigung. Wenn das zweite Computerprogramm exe2 im Rechenzentrum 2 ausgeführt wird, extrahiert dieses nicht nur die für die zweite Indikation ind2 benötigten Dateneinträge val1, sondern verarbeitet diese auch. In Figur 1 ist dies durch den Operator "%" dargestellt, sodass der Ausgabedatensatz 5 die verarbeiteten Dateneinträge x% und v% umfasst, die auf die ursprünglichen Werte x und v zurückzuführen sind. Der Schnitt des Verarbeitens ist beispielsweise ein Verrauschen, um die Dateneinträge weiter zu anonymisieren oder pseudonymisieren. Anschließend können diese verarbeiteten Dateneinträge im Ausgabedatensatz 5 gespeichert und an den zweiten Kommunikationspartner zurückgesandt werden.

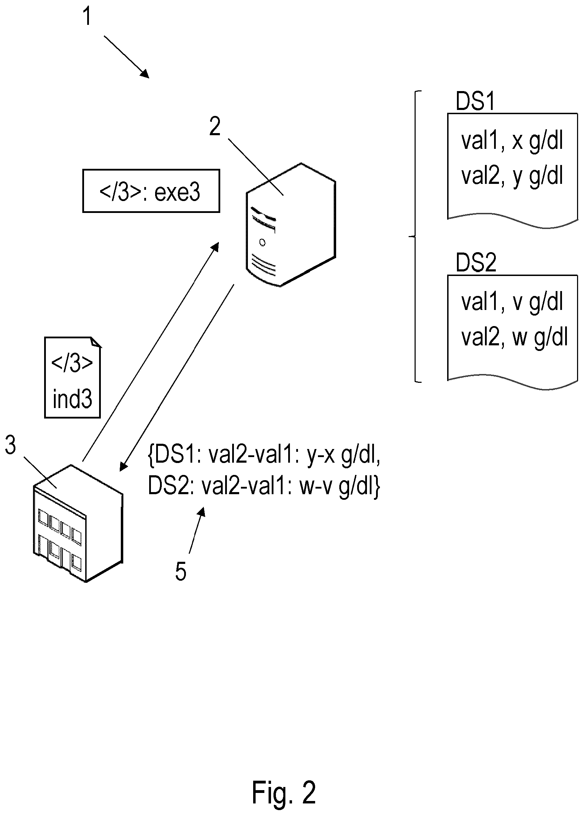

Figur 2 zeigt eine weitere Variante, in der alle Aspekte wie in Figur 1 beschrieben umgesetzt werden können, aber die folgenden beiden voneinander unabhängigen Varianten eingesetzt werden können. Erstens kann der erste Kommunikationsteilnehmer 3 statt einem bereits kompilierten Computerprogramm exe3 einen Quellcode </3> erzeugen und diesen an das Rechenzentrum 2 senden. Der Quellcode </3> enthält einen menschenlesbaren Text, der in einer Programmiersprache geschrieben ist und die logischen Anweisungen und Algorithmen enthält, die das Verhalten des Computerprogramms definieren. Der Quellcode </3> hat den Vorteil, dass alle Anweisungen und Algorithmen klar nachvollzogen werden können, was bei dem bereits kompilierten Computerprogramm exe3 nicht möglich wäre. Die Funktionsweise des Computerprogrammes exe3 ist damit leichter erkennbar, noch bevor es ausgeführt wird.

Der erste Kommunikationsteilnehmer 3 sendet daher den Quellcode </3> an das Rechenzentrum, das den Quellcode </3> empfängt und kompiliert oder interpretiert, um die darin definierten Anweisungen und Algorithmen auszuführen. Der Schritt des Kompilierens oder Interpretierens ist in Figur 2 durch die Funktion "</3> : exe3" dargestellt. Zuvor kann der Quellcode </3> jedoch überprüft werden, z.B. ob dieser nur jene Dateneinträge valj ausgibt oder heranzieht, die gemäß der Indikation ind1 zulässig sind (hierfür kann wieder die Liste 6 eingesetzt werden).

Die zweite Variation, die in Figur 2 dargestellt ist, ist eine weitere Art des Verarbeitens der Dateneinträge valj. Wie dargestellt werden jeweils zwei Dateneinträge val1, val2 desselben Datensatzes DSi verknüpft und in dieser Form im Ausgabedatensatz 5 gespeichert. Im Beispiel von Figur 2 ist der Schritt des Verarbeitens eine Differenz des zweiten Dateneintrags val2 mit dem ersten Dateneintrag val1 des jeweiligen Datensatzes DSi, siehe {DS1: val2-val1: y-x g/dl, DS2: val2-val1: w-v g/dl}. Statt zwei Dateneinträgen wurde somit nur ein Wert ausgegeben. Dies kann für Forschungszwecke vollkommen ausreichend sein, z.B. wenn diese Differenz das ausschlaggebende Kriterium zum Untersuchen der Indikation ist.

Im Allgemeinen können beim Verarbeiten von Dateneinträgen valj zumindest zwei (d.h. gegebenenfalls auch drei, vier, fünf, ...) Dateneinträge valj eines Datensatzes DSi herangezogen und aus diesen ein Wert berechnet werden, z.B. durch Differenzbildung, Addition, Multiplikation, Division, etc.

## Patentansprüche

1. Computerimplementiertes Verfahren zur anonymen Bereitstellung von sicherheitskritischen Datensätzen (DSi), insbesondere von gesundheitsbezogenen Datensätzen, umfassend die folgenden, in einem Rechenzentrum (2) durchgeführten Schritte:
- Speichern einer Vielzahl von sicherheitskritischen Datensätzen (DSi), die jeweils mehrere Dateneinträge (valj), die insbesondere medizinische Messdaten sind, umfassen,
- über eine externe Schnittstelle, Empfangen eines Computerprogramms (exe1, exe2, </3>), das dazu ausgebildet ist, bei dessen Ausführung ausgewählte Dateneinträge (valj) aus den Datensätzen (DSi) zu extrahieren und/oder zu verarbeiten, wobei die extrahierten oder verarbeiteten Dateneinträge (valj) in zumindest einem Ausgabedatensatz (5) gespeichert werden,
- Ausführen des Computerprogramms (exe1, exe2, </3>); und
- Ausgeben des zumindest einen Ausgabedatensatzes (5) über die genannte oder eine andere externe Schnittstelle.

2. Verfahren nach Anspruch 1, wobei das Computerprogramm (exe1, exe2, </3>) eine Indikation (ind1, ind2, ind3) einer zu untersuchenden Eigenschaft umfasst, insbesondere einer zu untersuchenden medizinischen Eigenschaft, wobei das Computerprogramm (exe1, exe2, </3>) dazu ausgebildet ist, im zumindest einen Ausgabedatensatzes nur für die genannte Indikation (ind1, ind2, ind3) relevante Daten zu speichern.

3. Verfahren nach Anspruch 2, wobei das Rechenzentrum (2) überprüft, ob der Sender des Computerprogramms (exe1, exe2, </3>) dazu berechtigt ist, Ausgabedatensätze (5) für die genannte Indikation (ind1, ind2, ind3) zu erhalten, und nur bei Vorliegen der Berechtigung den Ausgabedatensatz (5) ausgibt.

4. Verfahren nach Anspruch 2 oder 3, wobei das Rechenzentrum (2) überprüft, ob das Computerprogramm (exe1, exe2, </3>) nur auf solche Dateneinträge (valj) aus den Datensätzen (DSi) zugreift oder nur solche ausgewählten Dateneinträge (valj) aus den Datensätzen (DSi) in Ausgabedatensätzen (5) speichert, die zu der genannten Indikation (ind1, ind2, ind3) zugehörig sind, und nur in diesem Fall den Ausgabedatensatz (5) ausgibt.

5. Verfahren nach einem der Ansprüche 2 bis 4, wobei das Rechenzentrum (2) nur einen Zugriff auf jene Dateneinträge (valj) zulässt, die zu der genannten Indikation (ind1, ind2, ind3) zugehörig sind.

6. Verfahren nach einem der Ansprüche 2 bis 4, wobei im Rechenzentrum (2) eine Liste (6) hinterlegt ist, in welcher spezifiziert ist, ob der Sender des Computerprogramms (exe1, exe2, </3>) dazu berechtigt ist, Ausgabedatensätze (5) für eine jeweilige Indikation (ind1, ind2, ind3) zu erhalten; oder in welcher spezifiziert ist, welche Dateneinträge (valj) zu einer jeweiligen Indikation (ind1, ind2, ind3) zugehörig sind.

7. Verfahren nach einem der Ansprüche 1 bis 6, wobei der Schritt des Empfangens des Computerprogramms (exe1, exe2, </3>) folgende Schritte umfasst:
- Empfangen des Computerprogramms als Quellcode (</3>); und
- optional Kompilieren oder maschinelles Interpretieren des Quellcodes (</3>).

8. Verfahren nach einem der Ansprüche 2 bis 6 in Kombination mit Anspruch 6, umfassend den vor der Ausführung des Computerprogramms (</3>) durchgeführten Schritt:
- Überprüfen anhand des Quellcodes (</3>), ob das Computerprogramm dazu ausgebildet ist, bei dessen Ausführung nur solche Dateneinträge (valj) aus den Datensätzen (DSi) in Ausgabedatensätzen (5) zu speichern oder auf diese Dateneinträge zuzugreifen, die zu der genannten Indikation (ind1, ind2, ind3) zugehörig sind.

9. Verfahren nach einem der vorherstehenden Ansprüche, wobei beim Verarbeiten von Dateneinträgen (valj) zumindest zwei Dateneinträge (valj) eines Datensatzes (DSi) herangezogen werden und aus diesen ein Wert berechnet wird, der im Ausgabedatensatz (5) gespeichert wird.

10. Rechenzentrum (2), wobei das Rechenzentrum (2) dazu ausgebildet ist, das Verfahren der Ansprüche 1 bis 9 durchzuführen.
